# FASCICULE DE BREVET EUROPEEN

(11) **EP 2 536 463 B1**
(45) Date de publication et mention de la délivrance du brevet: **08.04.2020**
(21) Numéro de dépôt: 11703897.6
(22) Date de dépôt: 16.02.2011
(51) Int. Cl.: A61M 39/10, A61M 39/26

(54) **ENSEMBLE DE CONNECTEURS FACILEMENT NETTOYABLE POUR UN CIRCUIT LIQUIDE**
SET AUS EINFACH ZU REINIGENDEN STECKVERBINDERN FÜR EINEN FLÜSSIGKEITSKREISLAUF
SET OF EASILY CLEANABLE CONNECTORS FOR A LIQUID CIRCUIT

(30) Priorité: 09.06.2010 FR 1054539; 17.02.2010 FR 1051136
(43) Date de publication de la demande: 26.12.2012
(73) Titulaire: Vygon, 95440 Ecouen (FR)
(72) Inventeur: CARREZ, Jean-Luc, 95440 Ecouen (FR); GUYOMARC'H, Pierrick, 95120 Ermont (FR)
(74) Mandataire: Regimbeau
(86) Numéro de dépôt international: PCT/EP2011/052315
(87) Numéro de publication internationale: WO 2011/101389

(56) Documents cités:
- WO-A1-2006/078355
- WO-A2-2006/076656
- FR-A1- 2 931 363
- US-A1- 2005 090 805
- US-A1- 2007 017 583

## Description

L'invention concerne un connecteur pour un circuit de liquide, notamment à usage médical.

Il est connu du document EP 0 544 581 un tel connecteur sécurisé comportant un cône d'entrée distal de type Luer femelle destiné à être connecté avec un dispositif présentant un connecteur de type Luer mâle compatible et complémentaire. Le cône d'entrée distal est obturé par un septum ou membrane présentant une surface affleurante qui est facilement nettoyable pour enlever les germes ou bactéries avant de réaliser une éventuelle connexion. Le septum protège un tube qui, une fois la connexion réalisée, s'étend en partie dans le trou du connecteur de type Luer mâle qui lui est connecté. Une fois connecté, le connecteur du document EP 0 544 581 présente un passage tubulaire droit et un volume mort réduit. Le document EP 0 544 581 décrit un connecteur sécurisé femelle.

Toutefois, il devient alors nécessaire de disposer d'un connecteur mâle présentant les mêmes propriétés afin de bénéficier de la même sécurité d'utilisation que le connecteur femelle du document EP 0 544 581. D'autre part, l'utilisation d'un tel connecteur sécurisé mâle permettrait, en coopération avec le connecteur du document EP 0 544 581, de réaliser une connexion universelle sécurisée.

Il a donc été proposé, dans la demande de brevet français n° FR 2 931 363, un connecteur pour un circuit de liquide, notamment à usage médical, comprenant un raccord proximal, un raccord distal définissant un passage, un tube creux monté fixe sur le raccord proximal, s'étendant dans le passage du raccord distal et comportant une extrémité libre distale, et une membrane déformable élastiquement, sensiblement tubulaire, fermée à une extrémité distale par une épaisseur de membrane et destinée à recouvrir l'extrémité libre du tube creux de manière sensiblement étanche au repos, le raccord distal étant monté coulissant par rapport au raccord proximal entre une position distale de repos et une position proximale d'utilisation dans laquelle le tube creux peut être sélectivement dégagé de ladite épaisseur de membrane.

L'utilisation d'un tel raccord distal monté coulissant par rapport au raccord proximal permet notamment de dégager l'extrémité libre distale du tube creux protégée par la membrane afin de nettoyer celle-ci pour enlever tous germes ou bactéries avant connexion, le tube creux jouant le rôle de partie mâle du connecteur.

Néanmoins, la Demanderesse s'est rendu compte que les connecteurs mâle et femelle étaient difficilement nettoyables, notamment au niveau de leurs parties distales respectives comportant la membrane. Or, notamment dans le domaine de la chimiothérapie où les médicaments sont très agressifs à la fois lors de leur préparation par le personnel ou de leur injection aux patients, la propreté du matériel utilisé est fondamentale dans la mesure où les patients sont très sensibles aux maladies nosocomiales.

Par ailleurs, la connexion et la déconnexion de l'ensemble de connecteurs est difficile et nécessite plusieurs étapes de la part de l'opérateur.

Enfin, les moyens de verrouillage actuels ne permettent pas de garantir à l'utilisateur que les connecteurs sont effectivement bien connectés. En particulier, lorsque des filetages sont utilisés au niveau des extrémités des connecteurs mâle et femelle devant être connectées, l'opérateur ne peut souvent pas savoir s'il a suffisamment vissé les connecteurs ensemble et si la connexion des tubes est réalisée correctement.

Le document WO 2006/076656 décrit un premier raccord pour fluide pouvant se connecter à un deuxième raccord pour fluide en vue du transfert de fluides. Le premier raccord comporte un passage pour les fluides, un élément intérieur et une bague extérieure disposée autour de l'élément intérieur et pouvant au choix avancer ou reculer par rapport à lui. La bague extérieure peut être déplacée pour nettoyer l'élément intérieur. Pour faciliter le nettoyage, une partie distale de l'élément intérieur, peut être quasiment obturée lorsque le premier et le deuxième raccord sont déconnectés.

Le document US 2005/090805 décrit un ensemble de connexion qui peut être débranché soit manuellement soit automatiquement par l'application d'une force axiale suffisamment faible pour éviter de blesser le patient. Cet ensemble de connexion comprend un connecteur mâle et un connecteur femelle, comprenant chacun un tube de pénétration, monté dans un carter, et une membrane. Le connecteur mâle et le connecteur femelle comprennent en outre des moyens d'encliquetage associés afin de connecter leurs carters respectifs.

Le document FR 2 931 363, au nom de la Demanderesse, décrit un connecteur pour un circuit de liquide comprenant un raccord proximal, un raccord distal définissant un passage, un tube creux monté fixe sur le raccord proximal, s'étendant dans le passage du raccord distal et comportant une extrémité libre distale, et une membrane déformable élastiquement, sensiblement tubulaire, fermée à une extrémité distale par une épaisseur de membrane et destinée à recouvrir l'extrémité libre du tube creux de manière sensiblement étanche au repos. Le raccord distal est monté coulissant par rapport au raccord proximal entre une position distale de repos et une position proximale d'utilisation dans laquelle le tube creux peut être sélectivement dégagé de ladite épaisseur de membrane.

Le document WO 2006/078355 décrit un ensemble de connecteur comprenant un connecteur mâle Luer et un connecteur femelle Luer. Le connecteur mâle possède deux soupapes internes et une structure de génération de vide configurées pour faire revenir dans le connecteur mâle tout fluide restant sur l'interface entre les connecteurs pendant leur déconnexion et fermer de façon hermétique l'extrémité distale du connecteur mâle. Un membre élastique sollicite les soupapes mâles vers la position fermée et comprend un volume interne variable qui crée un vide lors de la disjonction des connecteurs. Les soupapes mâles sont réglées à une séquence spécifique correspondant à la fermeture d'une soupape de connecteur femelle interne de sorte que le vide généré par le connecteur mâle ait le plus grand effet de rappel de fluide.

Enfin, le document US 2007/017583 décrit un raccord Luer comprenant un boîtier présentant une première extrémité pourvue d'un embout Luer mâle et une seconde extrémité. Ce raccord comprend par ailleurs un élément obturateur rigide présentant une première extrémité ouverte et une seconde extrémité fermée, ainsi qu'un élément de retenue conçu pour coupler l'élément obturateur et le boîtier. Le boîtier comprend en outre un conduit rigide placé à l'intérieur du boîtier et se trouvant en communication fluidique avec la seconde extrémité du boîtier, ce conduit rigide étant adapté pour venir en prise avec la première extrémité ouverte de l'élément obturateur. Le boîtier contient un premier volume interne lorsque l'élément obturateur se trouve dans une première position et un second volume, inférieur au premier, lorsque l'élément obturateur se trouve dans une seconde position.

L'invention vise donc à proposer une connexion mâle et femelle améliorée.

Pour cela, l'invention propose un ensemble de connexion sécurisée pour un circuit liquide conforme à la revendication 1.

Certains aspects préférés mais non limitatifs de l'ensemble de connexion selon l'invention sont les suivants :
- la surface externe de la partie tubulaire du connecteur femelle est dépourvue de filetage,
- la surface externe de la partie tubulaire du connecteur mâle comprend un filetage de manière à constituer une entrée connectable de type "Luer-Lock" ,
- l'organe de connexion mâle et l'organe de connexion femelle sont des tubes creux, et en ce que le tube creux du connecteur mâle est adapté pour être inséré dans le tube creux du connecteur femelle,
- le moyen de retenue du moyen de verrouillage mâle comprend au moins un ergot s'étendant transversalement depuis la surface lisse par rapport à l'axe longitudinal du raccord,
- le moyen de verrouillage mâle comporte deux ergots s'étendant de part et d'autre du raccord, dans des directions opposées,
- le moyen de retenue du moyen de verrouillage femelle comprend au moins une gorge formée dans la surface d'une collerette et destinée à recevoir l'ergot du moyen de verrouillage mâle,
- la gorge est coudée,
- la collerette s'étend sur une partie seulement du raccord,
- les filetages s'étendent sur la surface du tube qui n'est pas recouverte par la collerette,
- la collerette est ajourée au niveau d'une extrémité proximale,
- la membrane comporte une fente dans l'épaisseur de membrane agencée de sorte à être traversée par l'organe de connexion en position amont de connexion.

Selon un deuxième aspect, l'invention propose l'utilisation d'un ensemble selon l'une des revendications 1 à 12, comprenant les étapes consistant à :
- nettoyer la membrane ;
- mettre en place les extrémités proximales des connecteurs mâles et femelles, de sorte que leurs membranes respectives soient en contact et que les deux connecteurs aient leurs axes longitudinaux confondus, afin de créer une jonction étanche des deux connecteurs ;
- insérer le connecteur mâle dans le connecteur femelle, de sorte que l'organe de connexion du connecteur mâle soit connecté dans l'organe de connexion du connecteur femelle ; et
- verrouiller l'ensemble.

Le connecteur pour un circuit liquide, notamment à usage médical, et présentant un axe longitudinal, comprend :
- un raccord proximal ;
- un raccord distal définissant un passage s'étendant le long de l'axe longitudinal ;
- un tube creux monté fixe sur le raccord proximal, s'étendant dans le passage du raccord distal et comportant une extrémité libre distale ; et
- une membrane déformable élastiquement, sensiblement tubulaire, fermée à une extrémité distale par une épaisseur de membrane et destinée à recouvrir l'extrémité libre du tube creux de manière sensiblement étanche au repos,
dans lequel surface du raccord distal est lisse et de section axiale invariante sur une partie substantiellement continue du raccord distal et comporte un moyen de retenue s'étendant transversalement, ladite surface lisse et ledit moyen de retenue formant un moyen de verrouillage mâle ou femelle.

Certains aspects préférés mais non limitatifs de ce connecteur sont les suivants :
- le moyens de retenue du moyen de verrouillage mâle comprend au moins un ergot s'étendant transversalement depuis la surface lisse par rapport à l'axe longitudinal du raccord distal ;
- le moyen de verrouillage mâle comporte deux ergots s'étendant de part et d'autre du raccord distal, dans des directions opposées ;
- le moyen de retenue du moyen de verrouillage femelle comprend au moins une gorge formée dans la surface d'une collerette et destinée à recevoir l'ergot du moyen de verrouillage mâle ;
- la gorge est coudée ;
- l'extrémité de la gorge servant de butée pour l'ergot comporte un renfoncement formant une retenue de l'ergot en position verrouillée ;
- la collerette s'étend sur une partie seulement du raccord distal ; et
- une partie centrale du raccord distal non recouverte par la collerette comprend des filetages de manière à constituer une entrée connectable de type "Luer-Lock.

Un ensemble d'au moins deux connecteurs conformes à ce qui précède, destinés à coopérer ensemble pour réaliser un circuit de fluides, notamment dans le domaine médical, présente le fait que le moyen de verrouillage de l'un des connecteurs est mâle, et le moyen de verrouillage de l'autre des connecteurs est femelle.

Certains aspects préférés mais non limitatifs d'un tel ensemble sont les suivants :
- l'un des moyens de verrouillage comporte au moins un ergot s'étendant transversalement depuis la surface lisse par rapport à l'axe longitudinal du raccord dista; et
- l'autre des moyens de verrouillage comporte une surface lisse (260) dans laquelle est formée au moins une gorge coudée destinée à recevoir l'ergot du premier moyen de verrouillage
- la surface lisse depuis laquelle s'étend l'ergot s'étend est la surface externe du raccord distal du moyen de verrouillage mâle ; et
- la surface lisse dans laquelle est formée la gorge est la surface interne d'une collerette) agencée sur l'extrémité distale du moyen de verrouillage femelle ;
- la largeur de la gorge est sensiblement égale au diamètre de l'ergot pris dans une direction transversale à une direction d'insertion de l'ergot dans la gorge, et la longueur de la gorge est sensiblement égale à la distance relative parcourue par les connecteurs lorsqu'ils sont couplés ; et
- les raccords distaux des deux connecteurs sont structurellement similaires, en dehors de leurs moyens de verrouillage respectifs, leurs membranes respectives affleurant à l'extrémité distales desdits raccords distaux de sorte que le couplage/découplage du connecteur mâle et du connecteur femelle est réalisé en un seul mouvement axial.

Une utilisation d'un tel ensemble comprend les étapes consistant à :
- nettoyer la membrane et les moyens de verrouillage respectifs des connecteurs ;
- mettre en place les extrémités distales des connecteurs, de sorte que leurs membranes respectives soient en contact et que les deux connecteurs aient leurs axes longitudinaux confondus, afin de créer une jonction étanche des deux connecteurs ;
- positionner angulairement les connecteurs, de sorte que l'au moins un ergot soit disposé dans l'axe longitudinal de la gorge correspondante;
- appuyer les deux connecteurs l'un contre l'autre jusqu'à ce que l'ergot bute contre l'extrémité de la gorge ; et
- tourner les connecteurs relativement l'un par rapport à l'autre de manière à verrouiller l'ergot dans la gorge.

D'autres caractéristiques, buts et avantages de la présente invention apparaîtront mieux à la lecture de la description détaillée qui va suivre, et en regard des dessins annexés donnés à titre d'exemples non limitatifs et sur lesquels :
La figure 1 est une vue tridimensionnelle d'un connecteur comprenant des moyens de verrouillage mâles selon l'invention, au repos ;
La figure 2 est une vue en coupe du connecteur de la figure 1 ;
Les figures 3a et 3b sont des vues tridimensionnelles de deux formes de réalisation d'un connecteur comprenant des moyens de verrouillage femelles selon l'invention, au repos ;
Les figures 4a et 4b sont des vues en coupe des connecteurs des figures 3a et 3b respectivement, au repos ;
La figure 5a est une vue de côté d'un ensemble de connecteurs selon l'invention correspondant aux figures 3a et 4a, mis en contact ;
La figure 5b est une vue de côté d'un ensemble de connecteurs selon l'invention correspondant aux figures 3b et 4b, mis en contact ;
La figure 6 est une vue en coupe de l'ensemble de la figure 5a.
La figure 7 est une vue en coupe de l'ensemble de la figure 5a, lorsque les connecteurs sont verrouillés ensemble ;
La figure 8 est une vue de côté de l'ensemble de la figure 7 ; et
La figure 9 est une vue en coupe d'une troisième forme de réalisation d'un en coupe d'un ensemble de connecteurs comprenant des moyens de verrouillage femelles selon l'invention.

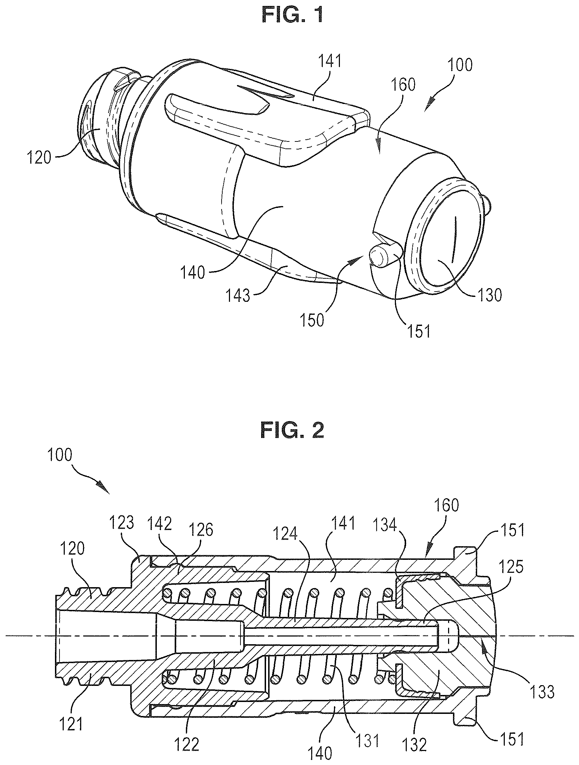

En référence aux figures 1 à 2, nous allons décrire un connecteur femelle 100 selon l'invention.

Le connecteur femelle 100 comporte un raccord proximal 120, une membrane 130 déformable élastiquement, sensiblement tubulaire, et un raccord distal 140.

Le raccord proximal 120 est de forme générale tubulaire et comporte une extrémité proximale qui comprend, ici, une entrée connectable 121 de type "Luer-Lock" femelle. En variante de réalisation, cette entrée connectable 121 est de type "Luer-Lock" mâle. Elle se prolonge, en direction distale, par un corps creux 122 qui comporte, au voisinage de l'extrémité proximale, un renflement 126 annulaire s'étendant en saillie et s'étendant sur une circonférence radialement externe du corps creux 122 du raccord proximal 120, destinée à coopérer avec une gorge annulaire complémentaire 142 prévue sur le raccord distal 140, afin de bloquer en translation les raccords proximal 120 et distal 140 l'un par rapport à l'autre, comme nous le verrons plus loin. Le corps creux 122 du raccord proximal 120 se termine, en direction distale, par un rebord 123, de forme circulaire, surmonté de manière coaxiale par un tube creux 124 qui s'étend en saillie du raccord proximal 120 en direction distale. Le tube creux 124 se termine par une extrémité libre 125 distale. Le tube creux forme un organe de connexion. Le raccord distal 140 est de forme tubulaire et définit un passage 141.

En variante, le raccord proximal 120 comporte au moins un picot, de préférence deux, qui coopèrent avec autant d'orifices prévu sur le raccord distal 140.

Nous allons maintenant décrire plus en détail la membrane 130 du connecteur 100.

Cette membrane 130 comporte des moyens élastiques 131, ici un ressort 131, par exemple du type ressort de compression hélicoïdal, formant un corps de membrane, s'étendant jusqu'à une extrémité distale 132 fermée par une épaisseur de membrane. Ces moyens élastiques 131 sont destinés à prendre appui sur le raccord proximal 120 et à venir pousser l'extrémité distale 132 de la membrane de manière à ce que cette dernière vienne dans une position avale stable d'obturation d'une extrémité distale du passage 141.

En variante, le corps de la membrane 130 est ondulé, et se présente sous la forme d'un soufflet.

Dans cette épaisseur de membrane est réalisée une fente 133 traversant ladite épaisseur de membrane.

Optionnellement, sur une circonférence radialement externe, l'extrémité distale 132 de la membrane 130 présente une gorge destinée à recevoir un moyen de renfort/striction 134, ici se présentant sous la forme d'une coupelle, qui entoure la fente 133 et permet d'assurer, en position de repos, que celle-ci est refermée de manière étanche pour éviter des éventuelles déperditions de fluides sous pression pouvant être présents dans le tube creux 124 lors d'une utilisation du connecteur 100 monté sur un circuit de fluides.

De plus, la forme particulière du corps 131 de membrane 130 permet à ce dernier de pouvoir se déformer de manière élastique dans un sens longitudinal et de faire office de ressort de rappel permettant à la membrane 130 de reprendre sa forme initiale de repos, telle qu'illustrée sur les figures 1 et 2, lors d'une déconnexion du connecteur 100.

Ainsi, le connecteur femelle 100 comporte une membrane 130 qui est encapsulée dans la partie tubulaire du raccord distal 140. Ceci permet, au surplus, en position avale stable d'obturation un serrage radial de la membrane 130, et en particulier de l'extrémité distale 132 de cette dernière, réalisé par une paroi de la partie tubulaire du raccord distal 140 : une meilleure étanchéité et une tenue en contre pression améliorée sont alors obtenues.

La membrane 130 est réalisée en un matériau élastomère, comme du polyisoprène, du silicone, ou encore un thermoplastique élastomère (TPE).

Lors d'un montage, le ressort 131 formant le corps de la membrane 130 est enfilé sur le tube creux 124 du raccord proximal 120.

Sur sa périphérie, de préférence en partie proximale, le raccord distal 140 comporte un moyen de verrouillage 150 mâle ou femelle. Ce moyen de verrouillage 150 sera détaillé plus loin dans la description.

Le raccord distal 140 comporte en outre des moyens de manipulation 143 du connecteur 100. Ici, ils sont au nombre de deux et s'étendent radialement en saillie de manière centrifuge de part et d'autre du raccord distal 140. Ces moyens de manipulation 143 permettent de maintenir fermement le connecteur 100 lors de sa connexion avec un connecteur mâle.

Lors d'un montage, le raccord distal 140 est monté sur le raccord proximal 120 de sorte que la saillie annulaire 126 vient se positionner dans la gorge annulaire 142 correspondante. Le raccord distal 140 est ensuite fixé selon les procédés usuels sur le raccord proximal 120, par exemple par collage de leurs surfaces de contact respectives, de manière à obtenir un carter monobloc, ou encore par soudure aux ultrasons.

Nous allons à présent décrire un connecteur mâle 200 selon l'invention.

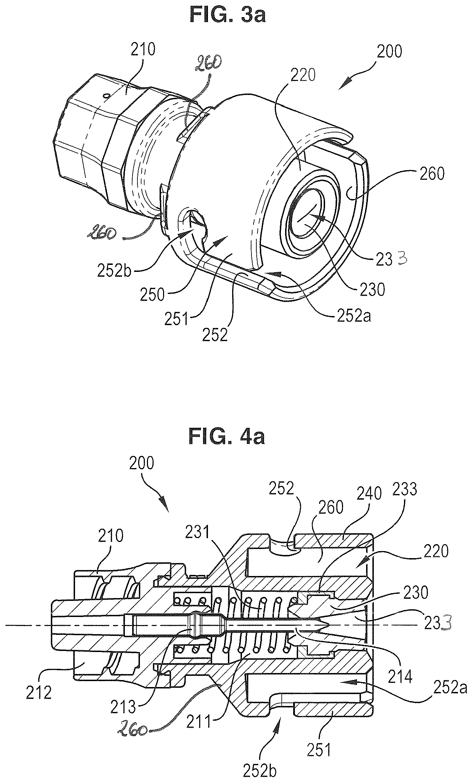

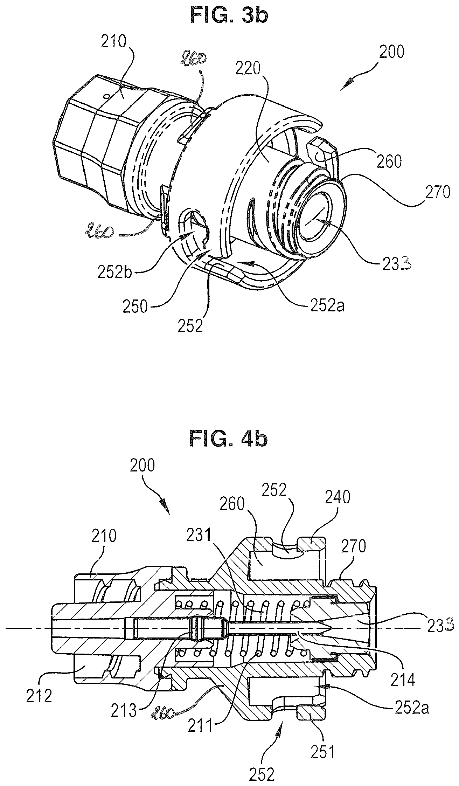

En référence aux figures 3a, 3b, 4a et 4b, un connecteur mâle 200 comporte un raccord proximal 210 et un carter 220 monté sur le raccord proximal 210. L'assemblage du carter 220 sur le raccord proximal 210 permet de délimiter une chambre sensiblement tubulaire 211 formant un passage. Le raccord proximal 210 comporte une entrée connectable 212, ici, de type "Luer-Lock" mâle. En variante de réalisation, cette entrée connectable 212 est de type "Luer-Lock femelle". Emmanché dans ce raccord proximal 210, le connecteur 200 comporte un organe de connexion 213, ici sous la forme d'un tube creux 213, s'étendant dans le passage sensiblement tubulaire 211 en direction distale.

Le carter 220 est formé d'un raccord distal 240 et comporte une partie tubulaire.

Le raccord proximal comporte une membrane ou septum 230 qui comprend une fente 233 dans son épaisseur. La fente 233 est sensiblement perpendiculaire à un axe longitudinal du connecteur 200.

Le connecteur 200 comporte enfin des moyens élastiques 231, ici un ressort 231, par exemple du type ressort de compression hélicoïdal, mais qui peut prendre également toute autre forme connue de l'homme du métier. Le ressort 231 est destiné à prendre appui sur le raccord proximal 210 et à venir pousser le septum 230 de manière à ce que ce dernier vienne dans une position avale stable d'obturation d'une extrémité distale du passage 211.

Une description plus détaillée d'un connecteur similaire au connecteur 200 est fournie dans le document EP 0 544 581 auquel il est possible de se référer à cette fin.

Le septum 230 est ici une membrane élastomérique de forme générale cylindrique de révolution comportant une fente 233 sensiblement plane et perpendiculaire à un axe longitudinal du septum 230. Cette fente 233 s'étend depuis une surface avale jusque dans une ouverture amont de forme sensiblement cylindrique de révolution destinée à recevoir une extrémité libre du tube creux 214 de manière étanche.

Le septum 230 est monté dans une coupelle rigide 234 formant des moyens de renfort/striction. En variante de réalisation, cette coupelle est surmontée d'un anneau déformable élastiquement, faisant partie de moyens de renfort/striction.

Une description plus détaillée des moyens de renfort/striction 234 est fournie dans le document FR 2 929 123 auquel il est possible de se référer à cette fin.

Ainsi, le connecteur mâle 200 comporte un septum (ou membrane) 230 qui est encapsulé dans la partie tubulaire du raccord distal 240. Ceci permet, au surplus, en position avale stable d'obturation un serrage radial du septum 230, et en particulier au niveau de la fente 233 de ce dernier, réalisé par une paroi de la partie tubulaire du raccord distal 240 : une meilleure étanchéité et une tenue en contre pression améliorée sont alors obtenues.

Sur sa périphérie, de préférence au niveau d'une extrémité distale, le raccord distal 240 comporte un moyen de verrouillage 250 femelle ou mâle.

Dans la suite de la description, nous décrirons plus en détail le mode de réalisation selon lequel le connecteur femelle 100 comporte un moyen de verrouillage mâle à baïonnette, tandis que le connecteur mâle 200 comporte un moyen de verrouillage femelle à baïonnette. Ceci n'est en aucun cas limitatif, dans la mesure où le mode de réalisation consistant à prévoir un moyen de verrouillage femelle sur le connecteur femelle 100 et un moyen de verrouillage mâle sur le connecteur mâle 200 est équivalent et également couvert par l'invention.

Le moyen de verrouillage 250 du connecteur mâle 200 est de préférence complémentaire du moyen de verrouillage 150 du connecteur femelle 100.

Selon l'invention, lorsque les moyens de verrouillage 250 sont femelles, ils sont formés d'une surface lisse 260 ayant une section axiale invariante sur une partie substantiellement continue du raccord distal 240, et d'un moyen de retenue 252 s'étendant transversalement.

Les moyens de verrouillage mâles 150 sont alors également constitués par une surface lisse 160 et présentent un moyen de retenue 151 s'étendant transversalement, de formes complémentaires aux formes de la surface lisse 260 et du moyen de retenue 252 des moyens de verrouillage femelles 250 respectivement.

Une telle forme de réalisation permet donc de faciliter le nettoyage des moyens de verrouillage, dans la mesure où elle réduit les arêtes saillantes sur les surfaces 160, 260 à nettoyer, par opposition aux moyens de verrouillage par filetage dont l'utilisation est répandue dans l'art antérieur.

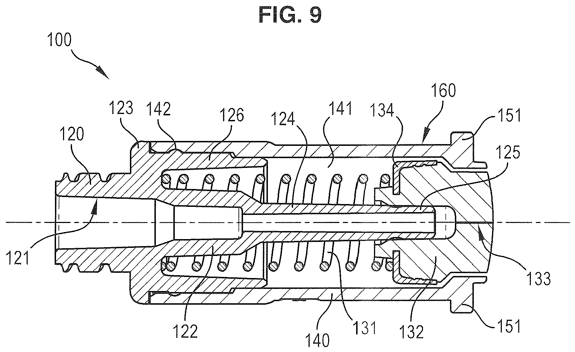

Répondent notamment à cette définition, et sont donc couverts par l'invention, les moyens de verrouillage à baïonnettes, par clipsage (i.e. par déformation élastique d'un élément de retenue faisant saillie depuis la surface de la partie distale d'un des moyens de verrouillage, puis pénétration dans un orifice complémentaire prévu dans la surface de la partie distale de l'autre des moyens de verrouillage, voir par exemple figure 9), etc.

Dans le cas des moyens de verrouillage à baïonnette, le moyen de retenue 151 du moyen de verrouillage mâle comprend au moins un ergot s'étendant transversalement vers l'extérieur depuis la surface externe lisse 260. De préférence, le moyen de retenue 151 comprend deux ergots, s'étendant de part et d'autre du raccord distal 140 du connecteur mâle 100.

Chaque ergot 151 peut avoir une section circulaire, carrée, elliptique, ou toute autre section adaptée pour maintenir efficacement en position le connecteur mâle 100 par rapport au connecteur femelle 200.

Le moyen de retenue du moyen de verrouillage femelle 250 quant à lui est alors constitué d'au moins une gorge 252 formée dans une surface 160 d'une collerette 251. La gorge 252 peut être traversante, pour alors former une fente comme illustré sur les figures annexées, ou borgne.

La collerette 251 est venue de matière avec le carter 220. En variante de réalisation, elle est rapportée sur le carter , par exemple par collage ou soudage ou encore pas tout autre moyen permettant un tel assemblage. Ici illustrée, la collerette 251 se présente sous la forme d'un tube relié, au niveau d'une extrémité proximale, par des nervures 260 au carter 220. Ainsi des passages 261 sont aménagés entre les nervures 261. Ici, les nervures 260 sont au nombre de quatre et uniformément réparties sur une circonférence de la collerette 251. En conséquence, les passages sont, eux-aussi au nombre de quatre et uniformément répartis sur ladite circonférence de la collerette 251. Une telle structure permet un nettoyage optimal de la collerette et donc du connecteur mâle 200.

De préférence, le moyen de verrouillage femelle 250 comprend au moins autant de gorges 252 qu'il y a d'ergots 151 sur le moyen de verrouillage mâle 150.

Chaque gorge 252 présente de préférence une extrémité libre débouchant sur une extrémité distale de la collerette 251, l'autre extrémité étant fermée et prévue pour former une butée longitudinale pour l'ergot 151.

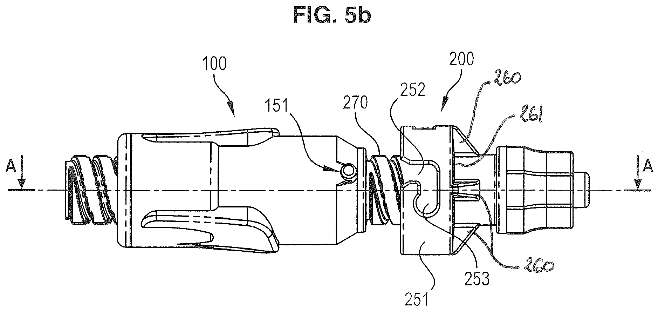

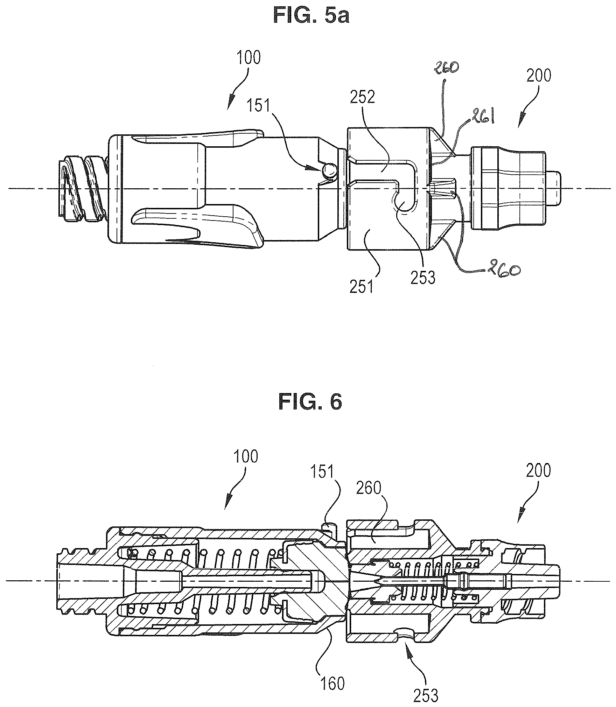

Par ailleurs, les gorges 252 sont agencées sur la collerette 251 de sorte que leur extrémité libre respective se trouve en regard d'un ergot 151 lorsque les extrémités distales 140, 240 des connecteurs mâle 200 et femelle 100 sont mises en contact avant connexion, comme illustré sur la figure 5.

De la sorte, lors de la connexion des connecteurs mâle 200 et femelle 100, chaque ergot 151 du moyen de verrouillage mâle 150 pénètre respectivement dans une gorge 252 du moyen de verrouillage femelle 250.

En variante (illustrée sur les figures 3b et 4b), la partie centrale 270 du raccord distal, entourée par la collerette, est filetée de manière à constituer une entrée connectable de type Luer-Lock femelle. Pour cela, la partie distale de la collerette 251 est raccourcie de sorte que la face de la partie centrale 270 soit accessible radialement. Ainsi, nous obtenons un moyen de verrouillage femelle pouvant être connecté soit à un moyen de verrouillage mâle 150 tel que celui décrit en référence à la figure 2, soit à un moyen de verrouillage de type "Luer-Lock" mâle complémentaire.

L'intérêt de cette forme de réalisation est qu'elle permet notamment la connexion du connecteur 200 avec une seringue comprenant un moyen de verrouillage de type Luer-Lock.

Cette forme de réalisation présente également l'avantage d'être facilement nettoyable dans la mesure où la surface interne 260 de la collerette 251 et la surface externe 160 comprenant les ergots 151 sont lisses, tandis que la partie filetée centrale 270 est accessible directement par un manipulateur qui souhaite la nettoyer.

Dans une autre variante, (non représentée sur les figures), le moyen de verrouillage femelle peut comporter une collerette comprenant au moins un ergot faisant saillie depuis sa surface interne, complémentaire d'un moyen de verrouillage mâle comprenant une surface externe lisse dans laquelle est formée une gorge (non traversante).

Avantageusement, ces formes de réalisations permettent de garantir à l'opérateur que les connecteurs mâle 200 et femelle 100 sont effectivement bien connectés.

En effet, l'opérateur peut vérifier visuellement et mécaniquement si l'ergot 151 est bien au fond de la gorge 252, ce qui implique que les tubes 124 et 215 sont effectivement bien connectés, contrairement aux connecteurs conventionnels comprenant des moyens de verrouillage filetés, qui ne permettent pas des telles vérifications.

La longueur d'une gorge 252 est de préférence au moins égale à la distance relative maximale parcourue par les connecteurs 100, 200 lorsqu'ils sont connectés par un opérateur. Cette distance relative sera davantage détaillée par la suite.

La largeur d'une gorge 252 est sensiblement égale au diamètre de l'ergot 151 correspondant, pris dans le sens transversal à la direction d'insertion de l'ergot 151 dans la gorge 252. De la sorte, l'ergot 151 peut se déplacer longitudinalement dans la gorge 252 tout en étant bloqué transversalement lors de son insertion dans la gorge 252.

Le cas échéant, la gorge 252 est en outre de forme coudée afin de maintenir l'ergot 151 fixe longitudinalement lorsque celui-ci arrive en butée. Par exemple, la gorge 252 présente une partie longitudinale 252a, de longueur au moins égale à la distance relative parcourue par les connecteurs 100, 200 lors de leur connexion, et une partie transversale 252b, s'étendant dans une direction sensiblement perpendiculaire à la direction d'insertion des ergots 151.

En variante, l'angle formé entre la partie transversale 252b et la partie longitudinale 252a de la gorge 252 est aigu, afin d'accentuer le maintien de l'ergot 151, et donc le verrouillage de l'ensemble des connecteurs 100, 200.

Néanmoins, quelle que soit la forme de réalisation, les gorges 252 formées dans une collerette 251 donnée sont identiques. En particulier, elles sont de taille, de forme et d'orientation identique, afin que les ergots 151 puissent facilement pénétrer dans la gorge 252 qui leur est associée.

En variante, les gorges 252 et ergots 151 associés peuvent être de largeur différentes deux à deux, de sorte qu'un ergot 151 donné (ayant un diamètre transversal donné) ne puisse pas pénétrer dans une gorge 252 ayant une largeur plus petite, pour assurer une fonction de détrompage.

Selon une forme de réalisation, l'extrémité fermée de chaque gorge 252 est en outre pourvue d'un renfoncement ou siège 253 disposé dans l'extrémité de la gorge 252 servant de butée à l'ergot 151. Le cas échéant, le renfoncement 253 est décalé par rapport à l'axe de la gorge 252, afin de bloquer transversalement l'ergot 151 dans la gorge 252, et donc d'améliorer encore davantage le verrouillage de l'ensemble de connecteurs 100, 200. Par exemple, lorsque la gorge 252 est coudée, comme illustré notamment sur les figures 4 et 5, le renfoncement 253 est disposé dans l'extrémité fermée de la partie transversale 252b de la gorge 252 et est décalé en direction de l'extrémité distale par rapport à l'axe de ladite partie transversale 252.

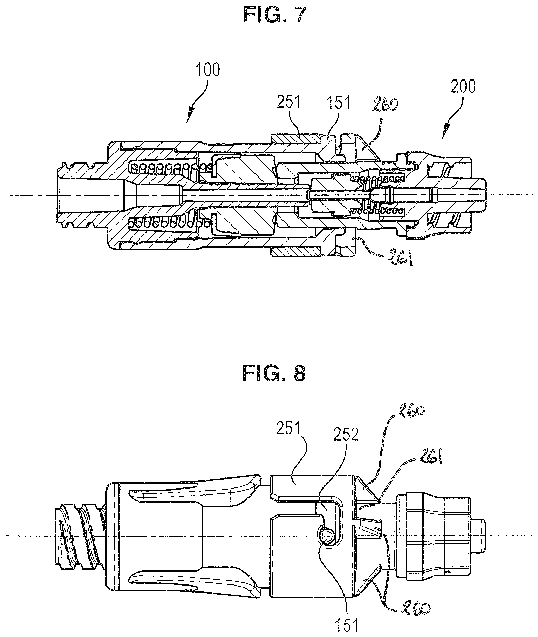

En référence aux figures 5 à 7, nous allons décrire les interactions des différents éléments qui viennent d'être décrits.

Les figures 5 et 6 représentent des connecteurs femelle 100 et mâle 200 dans une position avale stable d'obturation. Dans cette position, la membrane 130, respectivement 230, obture de manière étanche le passage 141, respectivement 211, de sorte qu'une surface avale de la membrane 130, respctivement, 230, affleure à l'extrémité avale dudit passage.

Pour information, dans cette position, un manipulateur du connecteur 100, 200 peut aisément nettoyer ladite surface avale de la membrane, afin de réduire les risques d'infections nosocomiales qui pourraient résulter d'un mauvais nettoyage ou d'un nettoyage difficile de cette surface.

Grâce aux moyens de verrouillage 150, 250 et à la structure des connecteurs mâle 200 et femelle 100 selon l'invention, l'étanchéité en contrepression est fortement augmentée, sans détruire les performances en termes de connexions/déconnexions répétées. Au surplus, l'utilisation de coupelles rigides 134, 234 empêche toute expulsion des septums 130, 230 en dehors des connecteurs 100, 200 selon l'invention sous l'effet de fortes pressions.

Lors d'une première utilisation, l'opérateur nettoie l'extrémité distale des membranes 130, 230 afin d'enlever toute présence de germes ou de bactéries, ainsi que les moyens de verrouillage à baïonnette 150, 250 (et le cas échéant la partie centrale 270).

Il est à noter d'ailleurs que le remplacement des filetages conventionnels par les moyens de verrouillage à baïonnette 150, 250 (et le cas échéant la partie centrale 270) selon l'invention facilite grandement ce nettoyage.

Une fois le nettoyage réalisé, l'opérateur met en contact les septums respectifs du connecteur 200 mâle et du connecteur 100 femelle, comme cela est illustré à la figure 5, puis connecte l'ensemble. Cette connexion entre le connecteur 100 et le connecteur 200 se fait en verrouillant les ergots 151 du raccord distal 140 du connecteur femelle 100 dans les gorges 252 correspondantes du raccord distal 240 du connecteur mâle 200.

Il est remarquable que, comme les raccords distaux 140 et 240 des deux connecteurs femelle 100 et mâle 200 sont structurellement similaires (en dehors bien entendu de leurs moyens de verrouillage respectifs), leurs membranes 130, 230 respectives affleurent au niveau de l'extrémité distale de leur raccord distal 140, 240. De la sorte, le couplage/découplage du connecteur mâle 200 et du connecteur femelle 100 n'est réalisé qu'en un seul mouvement axial. Il n'est en effet plus nécessaire de fixer les raccords distaux 140, 240 des connecteurs femelle 100 et mâle 200 avant de commencer à pousser les connecteurs l'un vers l'autre, ces deux opérations étant réalisées simultanément grâce, d'une part, à la structure des raccords distaux 140, 240, et d'autre part aux moyens de verrouillage conformes à l'invention.

Plus précisément, l'opérateur place les ergots 151 du connecteur femelle 100 en regard des gorges 252 correspondantes, puis fait glisser les connecteurs 100, 200 relativement l'un par rapport à l'autre en poussant le connecteur femelle 100, en prenant appui sur les moyens de manipulation 143 du raccord distal 140, vers le connecteur mâle 200.

Au cours de cette manipulation, l'extrémité distale du connecteur mâle 200 pousse sur l'extrémité distale de la membrane 130 du connecteur femelle 100, ce qui d'une part contraint le corps de membrane 131 à se comprimer, et d'autre part, force l'extrémité distale 125 du tube creux 124 à traverser, par le biais de la fente 133, l'épaisseur de membrane située à l'extrémité distale de la membrane 130.

Une fois que l'extrémité distale 125 du tube 124 a traversé l'épaisseur de membrane de l'extrémité distale de la membrane 130, cette extrémité distale 125 du tube 124 vient en contact d'appui avec le septum 230 du connecteur 200. L'extrémité distale 125 du tube 124 pousse ensuite le septum 230, d'une part, à l'encontre du ressort 231 sous-jacent et, d'autre part, à l'encontre du tube 214 qui traverse alors le septum 230 via la fente 233 qui y est pratiquée.

La poussée se poursuit jusqu'à ce que l'extrémité distale 125 du tube 124 du connecteur femelle 100 reçoive l'extrémité distale du tube 214 du connecteur mâle 200. Dès lors la connexion un passage de liquide sont réalisés entre les connecteurs mâle 200 et femelle 100.

La distance relative parcourue par les connecteurs 100, 200 au cours de la manipulation est définie par la longueur de la partie longitudinale 252a des gorges 252. Dans cette position finale, dans laquelle les connecteurs 100, 200 sont connectés et en communication fluidique, les ergots 151 sont donc en butée au fond de la partie longitudinale 252a des gorges 252.

L'opérateur fait alors tourner les connecteurs 100, 200 l'un par rapport à l'autre afin de positionner les ergots 151 dans l'extrémité fermée de la partie transversale 252b des gorges 252, jusqu'à atteindre leur siège 253, et verrouiller l'ensemble de connecteurs 100, 200.

A la déconnexion, l'opérateur fait tourner les connecteurs 100, 200 l'un par rapport à l'autre dans le sens contraire de celui du verrouillage, afin de sortir les ergots 151 de la partie transversale 252b des gorges 252, puis écarte les connecteurs 100, 200 avec l'aide de la poussée des deux ressorts. Les ergots 151 sortent alors de la partie longitudinale 252a des gorges 252, les membranes 130, 230 recouvrent de nouveau progressivement les tube 124, 214 respectivement de manière étanche et les membranes 130, 230 viennent obturer de manière étanche les extrémités distales respectives des connecteurs 100, 200.

Lors d'une connexion ou d'une déconnexion, l'étanchéité est assurée tout le long de l'opération. En effet, l'étanchéité est d'abord assurée par le contact de l'extrémité distale de la membrane 130 sur le septum 230, puis par le contact de l'extrémité distale 125 du tube creux 124 sur le septum 230 du connecteur mâle 200 - ledit contact étant maintenu par le verrouillage de l'ensemble de connecteurs - et enfin par l'insertion du tube 214 dans le tube creux 125.

Bien entendu, il est possible d'apporter à l'invention de nombreuses modifications sans sortir du cadre de celle-ci.

## Revendications

1. Ensemble de connexion sécurisée pour un circuit liquide, comprenant :
- un connecteur mâle (200) et un connecteur femelle (100), comprenant chacun un raccord proximal (120, 210) et un raccord distal (140, 240),
- le raccord distal (140, 240) de chacun des connecteurs (100, 200) définissant un passage (141, 211) et comprenant une partie tubulaire, dans laquelle s'étend de manière coaxiale un organe de connexion (124, 213) monté fixe sur le raccord proximal (120, 210) et une membrane (130, 230) déformable élastiquement, sensiblement tubulaire, fermée à une extrémité distale (132, 232) par une épaisseur de membrane et mobile entre une position aval d'obturation, dans laquelle la membrane (130, 230) recouvre une extrémité libre de l'organe de connexion (124, 213) de manière étanche, et une position amont de connexion, dans laquelle la membrane (130, 230) est traversée par l'organe de connexion (124, 213), la partie tubulaire du raccord distal (240) du connecteur mâle est adaptée pour être insérée, lors d'une connexion, dans la partie tubulaire (140) du raccord distal du connecteur femelle,
dans lequel, en position aval d'obturation, la membrane (130, 230) du connecteur femelle :
- est encapsulée dans la partie tubulaire du raccord distal (140, 240),
- affleure l'extrémité distale (132) du raccord distal (140, 240) et
- obture de manière étanche le passage (141, 211) dudit raccord distal (140, 240),
la surface externe (160, 260) de la partie tubulaire du raccord distal (140, 240) du connecteur femelle (100) étant lisse et de section axiale invariante sur une partie substantiellement continue et comportant un moyen de retenue (152, 252) s'étendant transversalement, ladite surface et ledit moyen de retenue (152, 252) formant un moyen de verrouillage mâle ou femelle,
l'ensemble de connexion étant **caractérisé en ce que** en position aval d'obturation, la membrane (130, 230) du connecteur mâle :
- est encapsulée dans la partie tubulaire du raccord distal (140, 240),
- affleure l'extrémité distale (132) du raccord distal (140, 240) et
- obture de manière étanche le passage (141, 211) dudit raccord distal (140, 240),
la surface externe (160, 260) de la partie tubulaire du raccord distal (140, 240) du connecteur mâle (100) étant lisse et de section axiale invariante sur une partie substantiellement continue et comportant un moyen de retenue (152, 252) s'étendant transversalement, ladite surface et ledit moyen de retenue (152, 252) formant un moyen de verrouillage mâle ou femelle.

2. Ensemble de connexion selon la revendication 1, **caractérisé en ce que** la surface externe (160) de la partie tubulaire du connecteur femelle (100) est dépourvue de filetage.

3. Ensemble de connexion selon l'une des revendications 1 et 2, dans lequel la surface externe (260) de la partie tubulaire du connecteur mâle (200) comprend un filetage de manière à constituer une entrée connectable de type "Luer-Lock".

4. Ensemble de connexion selon l'une des revendications 1 à 3, dans lequel l'organe de connexion mâle (213) et l'organe de connexion femelle (124) sont des tubes creux, et en ce que le tube creux (213) du connecteur mâle est adapté pour être inséré dans le tube creux (124) du connecteur femelle.

5. Ensemble de connexion selon l'une des revendications 1 à 4, dans lequel le moyen de retenue (252) du moyen de verrouillage mâle comprend au moins un ergot s'étendant transversalement depuis la surface lisse par rapport à l'axe longitudinal du raccord.

6. Ensemble de connexion selon l'une des revendications 1 à 5, dans lequel le moyen de verrouillage mâle (150) comporte deux ergots (152) s'étendant de part et d'autre du raccord, dans des directions opposées.

7. Ensemble de connexion selon l'une des revendications 1 à 6, dans lequel le moyen de retenue (252) du moyen de verrouillage femelle (250) comprend au moins une gorge formée dans la surface d'une collerette (251) et destinée à recevoir l'ergot (152) du moyen de verrouillage mâle (150).

8. Ensemble de connexion selon l'une des revendications 1 à 7, dans lequel la gorge (252) est coudée.

9. Ensemble de connexion selon la revendication 8, dans lequel la collerette (251) s'étend sur une partie seulement du raccord.

10. Ensemble selon les revendications 3 et 9 prises en combinaison, dans lequel les filetages s'étendent sur la surface du tube qui n'est pas recouverte par la collerette (251).

11. Ensemble de connexion selon l'une des revendications 8 à 10, dans lequel la collerette (251) est ajourée au niveau d'une extrémité proximale.

12. Ensemble de connexion selon l'une des revendications 1 à 11, **caractérisé en ce que** la membrane (130, 230) comporte une fente (133, 233) dans l'épaisseur de membrane agencée de sorte à être traversée par l'organe de connexion (124, 213) en position amont de connexion.

## Patentansprüche

1. Sichere Verbindungsanordnung für einen Flüssigkeitskreislauf, umfassend:
- einen männlichen Verbinder (200) und einen weiblichen Verbinder (100), die jeweils einen proximalen Anschluss (120, 210) und einen distalen Anschluss (140, 240) umfassen,
- wobei der distale Anschluss (140, 240) jedes der Verbinder (100, 200) einen Durchlass (141, 211) definiert und einen rohrförmigen Abschnitt aufweist, in dem sich koaxial ein Verbindungsglied (124, 213), das an dem proximalen Anschluss (120, 210) fest montiert ist, und eine elastisch verformbare, im Wesentlichen rohrförmige Membran (130, 230) erstreckt, die an einem distalen Ende (132, 232) durch eine Membrandicke verschlossen und zwischen einer stromabwärtigen Verschlussstellung, in der die Membran (130, 230) ein freies Ende des Verbindungsglieds (124, 213) dicht bedeckt, und einer stromaufwärtigen Verbindungsstellung beweglich ist, in der die Membran (130, 230) von dem Verbindungsglied (124, 213) durchquert wird, wobei der rohrförmige Abschnitt des distalen Anschlusses (240) des männlichen Verbinders dafür ausgelegt ist, bei einer Verbindung in den rohrförmigen Abschnitt (140) des distalen Anschlusses des weiblichen Verbinders eingeführt zu werden,
wobei die Membran (130, 230) des weiblichen Verbinders in der stromabwärtigen Verschlussstellung:
- in dem rohrförmigen Abschnitt des distalen Anschlusses (140, 240) eingekapselt ist,
- bündig mit dem distalen Ende (132) des distalen Anschlusses (140, 240) abschließt und
- den Durchlass (141, 211) des distalen Anschlusses (140, 240) dicht verschließt,
wobei die Außenfläche (160, 260) des rohrförmigen Abschnitts des distalen Anschlusses (140, 240) des weiblichen Verbinders (100) glatt ist und in einem im Wesentlichen kontinuierlichen Abschnitt einen unveränderlichen axialen Querschnitt aufweist und Rückhaltemittel (152, 252) umfasst, die sich in Querrichtung erstrecken, wobei die Oberfläche und die Rückhaltemittel (152, 252) männliche oder weibliche Verriegelungsmittel bilden,
wobei die Verbindungsanordnung **dadurch gekennzeichnet ist, dass** die Membran (130, 230) des männlichen Verbinders in der stromabwärtigen Verschlussstellung:
- in dem rohrförmigen Abschnitt des distalen Anschlusses (140, 240) eingekapselt ist,
- bündig mit dem distalen Ende (132) des distalen Anschlusses (140, 240) abschließt und
- den Durchlass (141, 211) des distalen Anschlusses (140, 240) dicht verschließt,
wobei die Außenfläche (160, 260) des rohrförmigen Abschnitts des distalen Anschlusses (140, 240) des männlichen Verbinders (100) glatt ist und in einem im Wesentlichen kontinuierlichen Abschnitt einen unveränderlichen axialen Querschnitt aufweist und Rückhaltemittel (152, 252) umfasst, die sich in Querrichtung erstrecken, wobei die Oberfläche und die Rückhaltemittel (152, 252) männliche oder weibliche Verriegelungsmittel bilden.

2. Verbindungsanordnung nach Anspruch 1, **dadurch gekennzeichnet, dass** die Außenfläche (160) des rohrförmigen Abschnitts des weiblichen Verbinders (100) kein Gewinde aufweist.

3. Verbindungsanordnung nach einem der Ansprüche 1 und 2, wobei die Außenfläche (260) des rohrförmigen Abschnitts des männlichen Verbinders (200) ein Gewinde aufweist, um einen anschließbaren Einlass vom Typ "Luer-Lock" zu bilden.

4. Verbindungsanordnung nach einem der Ansprüche 1 bis 3, wobei das männliche Verbindungsglied (213) und das weibliche Verbindungsglied (124) hohle Rohre sind und dass das hohle Rohr (213) des männlichen Verbinders dafür ausgelegt ist, in das hohle Rohr (124) des weiblichen Verbinders eingeführt zu werden.

5. Verbindungsanordnung nach einem der Ansprüche 1 bis 4, wobei die Rückhaltemittel (252) der männlichen Verriegelungsmittel zumindest einen Stift umfassen, der sich in Querrichtung von der glatten Oberfläche in Bezug auf die Längsachse des Anschlusses erstreckt.

6. Verbindungsanordnung nach einem der Ansprüche 1 bis 5, wobei die männlichen Verriegelungsmittel (150) zwei Stifte (152) umfassen, die sich von beiden Seiten des Anschlusses in entgegengesetzte Richtungen erstrecken.

7. Verbindungsanordnung nach einem der Ansprüche 1 bis 6, wobei die Rückhaltemittel (252) der weiblichen Verriegelungsmittel (250) zumindest eine Nut umfassen, die in der Oberfläche eines Bundes (251) ausgebildet und für die Aufnahme des Stiftes (152) der männlichen Verriegelungsmittel (150) bestimmt ist.

8. Verbindungsanordnung nach einem der Ansprüche 1 bis 7, wobei die Nut (252) gebogen ist.

9. Verbindungsanordnung nach Anspruch 8, wobei sich der Bund (251) nur über einen Teil des Anschlusses erstreckt.

10. Anordnung nach den Ansprüchen 3 und 9 in Kombination, wobei sich die Gewinde auf der Oberfläche des Rohrs erstrecken, die nicht durch den Bund (251) abgedeckt ist.

11. Verbindungsanordnung nach einem der Ansprüche 8 bis 10, wobei der Bund (251) an einem proximalen Ende mit Öffnungen versehen ist.

12. Verbindungsanordnung nach einem der Ansprüche 1 bis 11, **dadurch gekennzeichnet, dass** die Membran (130, 230) einen Schlitz (133, 233) umfasst, der derart in der Membrandicke angeordnet ist, dass er in der stromaufwärtigen Verbindungsstellung von dem Verbindungsglied (124, 213) durchquert wird.

## Claims

1. A secure connecter set for a liquid circuit, comprising:
- a male connector (200) and a female connector (100), each comprising a proximal coupler (120, 210) and a distal coupler (140, 240),
- the distal coupler (140, 240) of each of the connectors (100, 200) defining a passage (141, 211) and comprising a tubular part, in which a connection element (124, 213) fixedly mounted on the proximal coupler (120, 210) extends coaxially and an elastically deformable membrane (130, 230), substantially tubular, closed at a distal end (132, 232) by a membrane thickness and mobile between a downstream blocking position, in which the membrane (130, 230) tightly covers a free end of the connection element (124, 213), and an upstream connection position, in which the connection element (124, 213) passes through the membrane (130, 230), the tubular part of the distal coupler (240) of the male connector is adapted to be inserted, during connection, into the tubular part (140) of the distal coupler of the female connector,
wherein, in a downstream blocking position, the membrane (130, 230) of the female connector:
- is encapsulated in the tubular part of the distal coupler (140, 240),
- is flush with the distal end (132) of the distal coupler (140, 240) and
- tightly blocks the passage (141, 211) of said distal coupler (140, 240),
the outer surface (160, 260) of the tubular part of the distal coupler (140, 240) of the female connector (100) being smooth and having an unvarying axial cross-section over a substantially continuous part and comprising holding means (152, 252) extending transversally, said surface and said holding means (152, 252) together forming male or female locking means,
the connecter set being **characterized in that** in a downstream blocking position, the membrane (130, 230) of the male connector:
- is encapsulated in the tubular part of the distal coupler (140, 240),
- is flush with the distal end (132) of the distal coupler (140, 240) and
- tightly blocks the passage (141, 211) of said distal coupler (140, 240),
the outer surface (160, 260) of the tubular part of the distal coupler (140, 240) of the male connector (100) being smooth and having an unvarying axial cross-section over a substantially continuous part and comprising holding means (152, 252) extending transversally, said surface and said holding means (152, 252) together forming male or female locking means.

2. The connecter set as claimed in claim 1, **characterized in that** the outer surface (160) of the tubular part of the female connector (100) is devoid of threading.

3. The connecter set as claimed in any of claims 1 and 2, wherein the outer surface (260) of the tubular part of the male connector (200) comprises threading so as to constitute a connectable entry of "Luer-Lock" type.

4. The connecter set as claimed in any of claims 1 to 3, wherein the male connection element (213) and the female connection element (124) are hollow tubes, and in that the hollow tube (213) of the male connector is adapted to be inserted into the hollow tube (124) of the female connector.

5. The connecter set as claimed in any of claims 1 to 4, wherein the holding means (252) of the male locking means comprises at least one pin extending transversally from the smooth surface relative to the longitudinal axis of the coupler.

6. The connecter set as claimed in any of claims 1 to 5, wherein the male locking means (150) comprises two pins (152) extending on either side of the coupler, in opposite directions.

7. The connecter set as claimed in any of claims 1 to 6, wherein the holding means (252) of the female locking means (250) comprises at least one pin formed in the surface of a collar (251) and configured to receive the pin (152) of the male locking means (150).

8. The connecter set as claimed in any of claims 1 to 7, wherein the groove (252) is bent at an angle.

9. The connecter set as claimed in claim 8, wherein the collar (251) extends over only part of the coupler.

10. The set as claimed in claims 3 and 9 taken in combination, wherein the threading extend on the surface of the tube not covered by the collar (251).

11. The connecter set as claimed in any of claims 8 to 10, wherein the collar (251) is open-work at the level of a proximal end.

12. The connecter set as claimed in any of claims 1 to 11, **characterized in that** the membrane (130, 230) comprises a slot (133, 233) in the membrane thickness arranged so as to be traversed by the connection element (124, 213) in an upstream connection position.
